# FASCICULE DE BREVET EUROPEEN

(11) **EP 0 695 145 B1**
(45) Date de publication et mention de la délivrance du brevet: **05.08.1998**
(21) Numéro de dépôt: 94913662.6
(22) Date de dépôt: 18.04.1994
(51) Int. Cl.: A61B 17/58

(54) **IMPLANT POUR DISPOSITIF D'OSTEOSYNTHESE NOTAMMENT DU RACHIS**
IMPLANTAT FÜR OSTEOSYNTHESEVORRICHTUNG INSBESONDERE FÜR WIRBELSÄULE
IMPLANT FOR AN OSTEOSYNTHESIS DEVICE, IN PARTICULAR FOR THE SPINE

(30) Priorité: 19.04.1993 FR 9304584
(43) Date de publication de la demande: 07.02.1996
(73) Titulaire: STRYKER CORPORATION, Kalamazoo, MI 49002 (US); HENRY, Patrick, F-92200 Neuilly (FR); LAPRESLE, Philippe, F-92200 Neuilly-sur-Seine (FR); MISSENARD, Gilles, F-75016 Paris (FR)
(72) Inventeur: HENRY, Patrick, F-92200 Neuilly-sur-Seine (FR); LAPRESLE, Philippe, F-92200 Neuilly-sur-Seine (FR); MISSENARD, Gilles, F-75016 Paris (FR)
(74) Mandataire: Le Forestier, Eric
(86) Numéro de dépôt international: FR9400435
(87) Numéro de publication internationale: WO9423660

(56) Documents cités:
- EP-A- 0 509 322
- EP-A- 0 517 059
- FR-A- 2 677 874

## Description

La présente invention a trait au domaine des implants pour osthéosynthèse, notamment pour le rachis, et concerne plus particulièrement un implant pour dispositif d'ostéosynthèse notamment du rachis, comprenant une partie destinée à l'ancrage osseux et un corps dans lequel est formé un canal apte à recevoir une tige, le canal débouchant latéralement sur le corps, un élément creux apte à entourer le corps et possédant deux encoches aptes à recevoir la tige de part et d'autre du corps, et un moyen de fixation de l'élément creux sur le corps.

Le document EP-A-0 517 059 enseigne un implant de ce type.

Cet implant connu présente cependant un certain nombre d'inconvénients. Tout d'abord, il présente un encombrement relativement important du fait que le corps de l'implant possède vers l'arrière un prolongement fileté, le moyen de fixation consistant en un écrou de dimensions relativement importantes vissé sur ce prolongement. En outre, cet écrou possède par nature des angles saillants qui sont d'une manière générale indésirables pour les implants, car agressifs vis-à-vis de leur environnement.

En outre, du fait que le serrage de l'écrou doit en général s'effectuer avec un couple extrêmement important, il existe lors de ce serrage un risque d'arrachement et de libération de particules métalliques provenant des filets de l'écrou et/ou du prolongement fileté, et donc un risque de développement de métallose chez le patient.

La présente invention vise à pallier ou au moins atténuer tous ces inconvénients de la technique antérieure.

Elle propose à cet effet un implant du type mentionné en introduction, caractérisé en ce que :
l'élément creux est constitué par un capuchon coiffant le corps et possédant une partie de sommet arrondie dans laquelle est formée une ouverture,
le corps comporte en vis-à-vis de ladite ouverture un trou taraudé, et
le moyen de fixation comprend une vis dont une tête vient en appui sur la partie de sommet du capuchon et dont une partie filetée est vissée dans le trou taraudé.

Avantageusement, le canal présente, de l'extérieur vers le centre du corps, une inclinaison en direction de la partie d'ancrage osseux.

Dans une forme de réalisation préférée, le corps présente une partie de sommet de forme sensiblement hémisphérique et le capuchon présente une partie de sommet de forme sensiblement hémisphérique complémentaire; la tête de la vis présente alors préférentiellement une surface extérieure prolongeant avec continuité la surface arrondie du sommet du capuchon.

L'invention propose également dans le même esprit un implant pour dispositif d'ostéosynthèse notamment du rachis, comprenant une partie destinée à l'ancrage osseux et un corps dans lequel est formé un canal apte à recevoir une tige, le canal débouchant latéralement sur le corps, un élément creux fixé sur le corps pour immobiliser la tige dans le canal, et un moyen de fixation de l'élément creux sur le corps, caractérisé en ce que :
le canal présente, de l'extérieur vers le centre du corps, une inclinaison vers la partie d'ancrage osseux, et
l'élément creux coiffe le corps et possède une partie de sommet arrondie délimitée par un bord généralement droit en appui sur la tige de part et d'autre du canal pour bloquer ladite tige dans le canal, et une ouverture est formée dans ladite partie de sommet,
le corps comporte en vis-à-vis de ladite ouverture un trou taraudé, et
le moyen de fixation comprend une vis dont une tête vient en appui sur la partie de sommet de l'élément creux et dont une partie filetée est vissée dans le trou taraudé.

Le corps présente avantageusement une partie de sommet de forme sensiblement hémisphérique et l'élément creux consiste en une coupelle de forme sensiblement hémisphérique complémentaire.

La tête de la vis présente alors de préférence une surface extérieure prolongeant avec continuité la surface arrondie du sommet de la coupelle.

Enfin il est approprié que la vis soit montée de façon imperdable dans l'ouverture du capuchon.

D'autres aspects, buts et avantages de la présente invention apparaîtront mieux à la lecture de la description détaillée suivante de modes de réalisation préférés de celle-ci, donnée à titre d'exemple et faite en référence aux dessins annexés, sur lesquels:
la figure 1 est une vue en perspective éclatée d'un implant selon l'invention et d'une tige associée,
la figure 2 est une vue en perspective de l'implant et de la tige à l'état assemblé,
la figure 3 est une vue en élévation de côté d'une partie de l'implant, et
la figure 4 est une vue en élévation de côté d'un implant selon une variante de réalisation de l'invention.

En référence aux dessins, on a représenté un implant pour ostéosynthèse du rachis qui comprend un premier élément 10 qui comprend une partie 11 destinée à l'ancrage osseux et un corps 12 pour la fixation de l'élément 10 sur une tige T.

La partie d'ancrage 11 est ici réalisée sous la forme d'un crochet pédiculaire incurvé, dont le bord d'extrémité libre possède une encoche 11a, de façon classique en soi.

Le corps 12 réalisé d'un seul tenant avec le crochet 11 présente dans une région de base 12a un coutour général de forme cylindrique s'étendant à partir du crochet 11. Il se termine à l'opposé du crochet par une région de sommet 12b de forme généralement hémisphérique.

Dans la partie de base 12a du corps 12 est formé un canal 13 destiné à recevoir la tige T. Ce canal est ouvert dans une direction générale latérale et présente une hauteur légèrement supérieure au diamètre de la tige T.

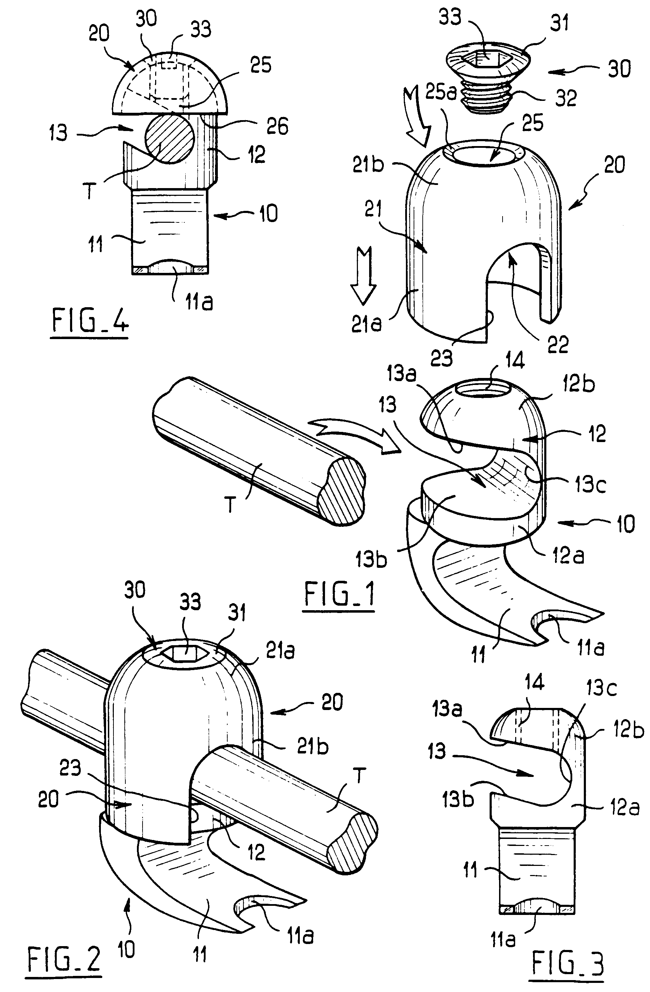

Comme on l'observe en particulier sur la figure 3, le canal 13 présente une inclinaison légèrement descendante de l'extérieur vers le centre du corps 12, et est délimité par deux parois respectivement supérieure et inférieure 13a et 13b, parallèles l'une à l'autre et donnant l'inclinaison précitée, et une paroi de fond 13c généralement semi-cylindrique.

Le canal s'étend sur toute la largeur du corps 12, et la tige T peut y être insérée latéralement jusqu'à son fond, avec une complémentarité de formes entre ce dernier et la tige.

Le corps 12 possède également un trou taraudé 14 qui s'étend suivant son axe vers le bas à partir du sommet de sa région 12b d'extrémité libre hémisphérique.

Dans le présent exemple, ce trou taraudé est traversant jusqu'à la paroi supérieure 13a du canal 13, mais il pourrait être en variante un trou borgne ouvert seulement au sommet du corps 12.

L'implant selon l'invention comprend en outre un capuchon 20 apte à venir coiffer le corps 12 dans le canal 12a duquel la tige T a été mise en place. Ce capuchon présente une paroi 21 dont une région de base 21a est de contour général cylindrique de révolution et dont une région de sommet 21b présente un contour général hémisphérique. Le capuchon 20 définit une cavité intérieure 22 dont la forme, au moins en section transversale, est identique à la forme extérieure du corps 12, de manière à pouvoir coiffer ce dernier avec un jeu transversal aussi réduit que possible, sans toutefois compromettre un glissement aisé entre le corps 12 et le capuchon 20 lors de la mise en place de ce dernier.

La partie de paroi 21a présente dans deux régions diamétralement opposées respectivement deux encoches en forme générale de "U", respectivement 23, dont une seule est visible sur les figures 1 et 2, orientées parallèlement à l'axe du capuchon (vertical sur les figures). Chaque encoche s'étend vers le haut à partir du bord inférieur de la paroi 21a, présente une largeur légèrement supérieure au diamètre de la tige T et se termine à une hauteur bien déterminée par un fond semi-circulaire, pour une complémentarité de formes avec le dessus de la tige.

Au sommet du capuchon 20 est formée une ouverture 25 d'un diamètre égal ou légèrement supérieur au diamètre du trou taraudé 14 du corps 12. Cette ouverture présente un biseau périphérique noté 25a.

L'implant selon l'invention comprend enfin une vis 30 qui comporte une tête 31 et une partie filetée 32 complémentaire du taraudage formé dans le corps 12.

La tête 31 possède une surface inférieure, autour de la partie 32, qui est biseautée pour s'adapter au biseau 25a du capuchon.

La surface supérieure de la tête 31 est en forme de portion de sphère ayant approximativement même rayon que la surface sphérique externe de la partie de sommet 21b du capuchon. De cette manière, après vissage, la tête 31 peut venir en affleurement avec la surface externe de la partie de sommet 21b du capuchon.

Par ailleurs, il est avantageux que le filetage de la vis 30 et le diamètre de l'ouverture 25 soient conçus de manière à assurer un maintien de la vis 30 à l'état fou dans ladite ouverture avant l'assemblage, pour rendre ladite vis imperdable. Ceci s'effectue avantageusement en donnant à l'ouverture 25 un diamètre légèrement inférieur au diamètre extérieur hors-tout des filets de la vis.

On simplifie notablement de la sorte la manipulation des diverses parties de l'implant par le chirurgien.

Dans cette surface supérieure est formé un aménagement de manoeuvre et de serrage, en l'espèce une cavité 33 à six pans hexagonaux destinée à recevoir l'extrémité d'une clé de type "Allen".

Les divers éléments de l'implant tel que décrit ci-dessus sont bien entendu réalisés en un matériau biocompatible tel qu'un alliage de titane ou l'acier inoxydable.

La pose de l'implant tel que décrit ci-dessus s'effectue de la manière suivante : tout d'abord, la partie d'ancrage 11 est placée sur la vertèbre de la manière usuelle, de manière à positionner le corps 12 à l'emplacement voulu. La tige T est ensuite engagée latéralement dans le canal 13, jusqu'au fond de celui-ci. Il est important d'observer ici que la forme hémisphérique du sommet 12b du corps 12 et l'inclinaison du canal 13 comme indiqué plus haut permettre de rendre cet engagement plus facile notamment dans le cas où la tige T a déjà été solidarisée avec un ou plusieurs autres implants. En effet, si l'on amène la tige T au voisinage du sommet de l'implant, et si on la presse vers le bas du côté de l'entrée du canal 13, la tige va glisser progressivement, sans obstacle, le long du côté du corps 12 puis pénétrer facilement dans le canal 13 dès qu'elle arrivera au droit de celui-ci.

Ensuite, le capuchon 20 est mis en place de manière à coiffer le corps 12 de l'élément 10 et la tige T, cette dernière pénétrant au niveau des deux sorties diamétralement opposées du canal dans les deux encoches 23, qui ont été préalablement convenablement orientées.

On observera qu'à partir de ce moment, le capuchon 20 est bloqué en rotation par la coopération de la tige T avec les encoches 23.

La vis est alors engagée dans l'ouverture 25 et vissée dans le taraudage 14 à l'aide d'un outil approprié, pour solidariser le corps 12 et le capuchon 20 et emprisonner fermement la tige T. On observera ici que le fond de chaque encoche 23 est positionné de manière à venir en appui contre la tige T avant que le fond de la cavité 22 du capuchon 20 ne viennent en appui contre le dôme 120 du corps 12, de manière à assurer un blocage effectif de la tige.

L'implant après assemblage et blocage est représenté sur la figure 2. On observe qu'il n'existe pratiquement aucun angle saillant vers l'extérieur. En particulier, la tête de la vis 30 prolonge avec continuité la surface en dôme du capuchon. On observe également que les risques d'arrachement de particules de matière lors du serrage de la vis avec couple élevé n'existent pratiquement que dans des régions intérieures de l'implant, c'est-à-dire au niveau du trou taraudé 14, ce qui réduit les risques de métallose.

La figure 4 illustre une variante de réalisation de l'invention. Selon cette variante, le capuchon 20 présente seulement une paroi généralement hémisphérique complémentaire de la région hémisphérique 120 du corps, et est délimitée en direction de la partie d'ancrage 11 par un bord droit annulaire 26.

Lors du serrage du capuchon 20 sur le corps 12 à l'aide de la vis 30, comme décrit plus haut, le bord 26 vient prendre appui sur la tige T de part et d'autre du canal 13 pour bloquer ladite tige. Dans cette variante, une inclinaison substantielle du canal, comme illustré, est requise pour assurer un blocage satisfaisant, car le capuchon 20 ne comporte plus les encoches 23 des figures 1 à 3 et c'est seulement le bord 26 qui s'oppose à un mouvement de la tige T hors de son canal 13. Et cette opposition est d'autant plus efficace que le bord 26 est incliné par rapport à la direction d'un mouvement de la tige hors du canal.

Bien entendu, la présente invention n'est nullement limitée aux formes de réalisation décrites ci-dessus et représentées sur les dessins, mais l'homme de l'art saura y apporter toute variante ou modification conforme à son esprit.

En particulier, bien que la partie d'ancrage osseux 11 ait été décrite comme étant un crochet, il peut s'agir également, entre autres, d'une vis telle qu'une vis pédiculaire.

## Revendications

1. Implant pour dispositif d'ostéosynthèse notamment du rachis, comprenant une partie (11) destinée à l'ancrage osseux et un corps dans lequel est formé un canal (13) apte à recevoir une tige (T), le canal débouchant latéralement sur le corps, un élément creux (20) apte à entourer le corps (12) et possédant deux encoches aptes à recevoir la tige de part et d'autre du corps (12), et un moyen (30) de fixation de l'élément creux sur le corps, caractérisé en ce que :
l'élément creux est constitué par un capuchon (20) coiffant le corps et possédant une partie de sommet arrondie (21b) dans laquelle est formée une ouverture (25),
le corps (12) comporte en vis-à-vis de ladite ouverture un trou taraudé (14), et
le moyen de fixation comprend une vis (30) dont une tête (31) vient en appui sur la partie de sommet (21b, 25a) du capuchon et dont une partie filetée (32) est vissée dans le trou taraudé.

2. Implant selon la revendication 1, caractérisé en ce que le canal (13) présente, de l'extérieur vers le centre du corps (12), une inclinaison en direction de la partie (11) d'ancrage osseux.

3. Implant selon la revendication 1 ou 2, caractérisé en ce que le corps présente une partie de sommet (12b) de forme sensiblement hémisphérique et le capuchon présente une partie de sommet (21b) de forme sensiblement hémisphérique complémentaire.

4. Implant selon la revendication 1, 2 ou 3, caractérisé en ce que la tête (31) de la vis présente une surface extérieure prolongeant avec continuité la surface arrondie du sommet (21b) du capuchon.

5. Implant selon l'une des revendications 1 à 4, caractérisé en ce que la vis (30) est montée de façon imperdable dans l'ouverture (25) du capuchon.

6. Implant pour dispositif d'ostéosynthèse notamment du rachis, comprenant une partie (11) destinée à l'ancrage osseux et un corps dans lequel est formé un canal (13) apte à recevoir une tige (T), le canal débouchant latéralement sur le corps, un élément creux (20) fixé sur le corps (12) pour immobiliser la tige dans le canal (13), et un moyen (30) de fixation de l'élément creux sur le corps, caractérisé en ce que :
le canal (13) présente, de l'extérieur vers le centre du corps, une inclinaison vers la partie d'ancrage osseux, et
l'élément creux (20) coiffe le corps et possède une partie de sommet arrondie délimitée par un bord généralement droit (26) en appui sur la tige (T) de part et d'autre du canal (13) pour bloquer ladite tige dans le canal, et une ouverture (25) est formée dans ladite partie de sommet,
le corps comporte en vis-à-vis de ladite ouverture un trou taraudé (14), et
le moyen de fixation comprend une vis (30) dont une tête (31) vient en appui sur la partie de sommet de l'élément creux et dont une partie filetée (32) est vissée dans le trou taraudé.

7. Implant selon la revendication 6, caractérisé en ce que le corps (12) présente une partie de sommet (12b) de forme sensiblement hémisphérique et l'élément creux (20) consiste en une coupelle de forme sensiblement hémisphérique complémentaire.

8. Implant selon la revendication 6 ou 7, caractérisé en ce que la tête (31) de la vis présente une surface extérieure prolongeant avec continuité la surface arrondie du sommet de la coupelle (20).

9. Implant selon l'une des revendications 6 à 8, caractérisé en ce que la vis (30) est montée de façon imperdable dans l'ouverture (25) de l'élément creux.

## Claims

1. An implant for an ostheosynthesis device, in particular for the spine, the device comprising an anchoring portion (11) for anchoring to bone and a body in which a channel (13) is formed suitable for receiving a rod (T), the channel opening out sideways in the body, a hollow element (20) suitable for surrounding the body (12) and possessing two notches suitable for receiving the rod on either side of the body (12), and means (30) for fixing the hollow element on the body, the implant being characterized in that:
the hollow element is constituted by a cover (20) fitting over the body and having a rounded top portion (21b) in which an opening (25) is formed;
the body (12) including a tapped hole (14) facing said opening; and
the fixing means comprising a screw (30) having a head (31) that bears against the top portion (21b, 25a) of the cover and a threaded portion (32) that is screwed in the tapped hole.

2. An implant according to claim 1, characterized in that going from the outside towards the center of the body (12), the channel (13) slopes towards the bone anchoring portion (11).

3. An implant according to claim 1 or 2, characterized in that the body has a top portion (12b) that is substantially hemispherical in shape and the cover has a top portion (21b) that is of complementary substantially hemispherical shape.

4. An implant according to claim 1, 2, or 3, characterized in that the head (31) of the screw has an outside surface that extends the rounded top surface (21b) of the cover with continuity.

5. An implant according to any one of claims 1 to 4, characterized in that the screw (30) is mounted in unloseable manner in the opening (25) of the cover.

6. An implant for an ostheosynthesis device, in particular for the spine, the device comprising an anchoring portion (11) for anchoring to bone and a body in which a channel (13) is formed suitable for receiving a rod (T), the channel opening out sideways in the body, a hollow element (20) fixed on the body (12) to hold the rod in the channel (13), and means (30) for fixing the hollow element on the body, the implant being characterized in that:
going from the outside towards the center of the body, the channel (13) slopes towards the bone anchoring portion; and
the hollow element (20) fits over the body and possesses a rounded top portion terminated by a generally flat edge (26) bearing against the rod (T) at either end of the channel (13) to lock said rod in the channel, an opening (25) being formed in said top portion;
the body including a tapped hole (14) facing said opening; and
the fixing means comprising a screw (30) having a head (31) that bears against the top portion (21b, 25a) of the hollow element and a threaded portion (32) that is screwed in the tapped hole.

7. An implant according to claim 6, characterized in that the body has a top portion (12b) that is substantially hemispherical in shape and the hollow element (20) comprises a cap that is of complementary substantially hemispherical shape.

8. An implant according to claim 6 or 7, characterized in that the head (31) of the screw has an outside surface that extends the rounded top surface of the cap (20) with continuity.

9. An implant according to any one of claims 6 to 8, characterized in that the screw (30) is mounted in unloseable manner in the opening (25) of the hollow element.

## Patentansprüche

1. Implantat für eine Osteosyntheseeinrichtung, insbesondere für die Wirbelsäule, mit einem Abschnitt (11), der zur Knochenverankerung bestimmt ist, und einem Körper, in dem ein Kanal (13) ausgebildet ist, der dazu eingerichtet ist, eine Stange (T) aufzunehmen, wobei der Kanal seitlich am Körper mündet, einem hohlen Element (20), das dazu eingerichtet ist, den Körper (12) zu umgeben, und zwei Auskerbungen besitzt, die dazu eingerichtet sind, die Stange beiderseits des Körpers (12) aufzunehmen, und einem Mittel (30) zur Befestigung des hohlen Elements auf dem Körper,
dadurch gekennzeichnet, daß
das hohle Element von einer Kappe (20) gebildet ist, die den Körper bedeckt und einen abgerundeten Scheitelabschnitt (21b) besitzt, in dem eine Öffnung (25) ausgebildet ist,
der Körper (12) gegenüber der genannten Öffnung eine Gewindebohrung (14) aufweist, und
das Befestigungsmittel eine Schraube (30) aufweist, von der ein Kopf (31) in Anlage auf dem Scheitelabschnitt (21b, 25a) der Kappe gelangt und von der ein Gewindeabschnitt (32) in die Gewindebohrung eingeschraubt ist.

2. Implantat nach Anspruch 1, dadurch gekennzeichnet, daß der Kanal (13) von außen zur Mitte des Körpers (12) eine Neigung in Richtung des Knochenverankerungsabschnitts (11) aufweist.

3. Implantat nach Anspruch 1 oder 2, dadurch gekennzeichnet, daß der Körper einen im wesentlichen halbkugelförmigen Scheitelabschnitt (12b) aufweist und daß die Kappe einen Scheitelabschnitt (21b) mit einer im wesentlichen halbkugeligen komplementären Form aufweist.

4. Implantat nach Anspruch 1, 2 oder 3, dadurch gekennzeichnet, daß der Kopf (31) der Schraube eine Außenfläche aufweist, die kontinuierlich die abgerundete Scheitelfläche (21b) der Kappe verlängert.

5. Implantat nach einem der Ansprüche 1 bis 4, dadurch gekennzeichnet, daß die Schraube (30) unverlierbar in der Öffnung (25) der Kappe angebracht ist.

6. Implantat für eine Osteosyntheseeinrichtung, insbesondere für die Wirbelsäule, mit einem Abschnitt (11), der zur Knochenverankerung bestimmt ist, und einem Körper, in dem ein Kanal (13) ausgebildet ist, der dazu eingerichtet ist, eine Stange (T) aufzunehmen, wobei der Kanal seitlich am Körper mündet, einem hohlen Element (20), das am Körper (12) befestigt ist, um die Stange im Kanal (13) festzulegen, und Mitteln (30) zur Befestigung des hohlen Elements auf dem Körper, dadurch gekennzeichnet, daß
der Kanal (13) von außen zur Mitte des Körpers hin eine Neigung zum Knochenverankerungsabschnitt hin aufweist, und
das hohle Element (20) den Körper abdeckt und einen abgerundeten Scheitelabschnitt besitzt, der durch einen im wesentlichen geraden Rand (26) begrenzt ist, der beiderseits des Kanals (13) auf der Stange (T) aufsitzt, um die genannte Stange im Kanal festzulegen, und daß eine Öffnung (25) im genannten Scheitelabschnitt ausgebildet ist,
der Körper gegenüber der genannten Öffnung eine Gewindebohrung (14) aufweist, und
das Befestigungsmittel eine Schraube (30) aufweist, von der ein Kopf (31) in Anlage auf dem Scheitelabschnitt des hohlen Elements gelangt und von der ein Gewindeabschnitt (32) in die Gewindebohrung eingeschraubt ist.

7. Implantat nach Anspruch 6, dadurch gekennzeichnet, daß der Körper (12) einen im wesentlichen halbkugelförmigen Scheitelabschnitt (12b) aufweist und das hohle Element (20) aus einer Schale mit einer im wesentlichen halbkugeligen komplementären Form besteht.

8. Implantat nach Anspruch 6 oder 7, dadurch gekennzeichnet, daß der Kopf (31) der Schraube eine Außenoberfläche aufweist, die kontinuierlich die abgerundete Oberfläche des Scheitels der Schale (20) verlängert.

9. Implantat nach einem der Ansprüche 6 bis 8, dadurch gekennzeichnet, daß die Schraube (30) unverlierbar in der Öffnung (25) des hohlen Elements angebracht ist.
